# EUROPEAN PATENT APPLICATION

(11) **EP 2 338 497 A1**
(43) Date of publication of application: **29.06.2011**
(21) Application number: 11152667.9
(22) Date of filing: 12.08.2002
(51) Int. Cl.: A61K 31/70, A61K 31/715

(54) **Treatment and prevention of heat shock protein-associated diseases and conditions**

(30) Priority: 10.08.2001 US 311325 P
(62) Divisional of application: 02757067.0
(71) Applicant: Eisai R&D Management Co., Ltd., Tokyo 112-8088 (JP)
(72) Inventor: Kobayashi, Seiichi, Belmont, MA 02478 (US); Zhang, Minghuang, Windham, NH 03087 (US); Shirota, Hiroshi, Belmont, MA 02478 (US)
(74) Representative: HOFFMANN EITLE

(57) **Abstract**

The invention provides methods of treating and preventing heat shock protein-associated diseases and conditions.

## Description

### Field of the Invention

This invention relates to methods of treating and preventing heat shock protein-associated diseases and conditions.

### Background of the Invention

Lipopolysaccharide (LPS), which is produced by all gram-negative bacteria, is harmful to mammals because it stimulates the release of macrophage inflammatory mediators and initiates multiple inflammatory events that cause host tissue damage. LPS has three main regions: a long chain polysaccharide (the O antigen), a core region, and a Lipid A region. A synthetic Lipid A analog, designated E5564 and described in U.S. Patent No. 5,935,938, has been shown to inhibit the stimulating activity of LPS on macrophages *in vitro* and to suppress the pathological effects of LPS *in vivo*. Because of the superior activity and good physiochemical properties of E5564, a phase II clinical trial of this drug is ongoing in patients with sepsis.

Human heat shock protein (HSP) 60, a dominant antigen that is involved in the pathogenesis of many inflammatory diseases, including inflammatory bowel disease (IBD), arthritis, asthma, and atherosclerosis, has recently been implicated in macrophage activation. Like LPS, HSP60 stimulates macrophage cytokine synthesis via a Toll-like receptor (TLR)4-dependent pathway. Humans express heat shock proteins in addition to HSP60, such as HSP70 and HSP40. Also, many other species, including pathogenic bacteria, express their own heat shock proteins under conditions of stress.

### Summary of the Invention

The invention provides methods of treating or preventing non-sepsis, Toll-like Receptor (TLR)-mediated (e.g., TLR4 or TLR2-mediated), heat shock protein-associated diseases or conditions in patients. These methods involve administering to patients an effective amount of a Lipid A analog of the formula: 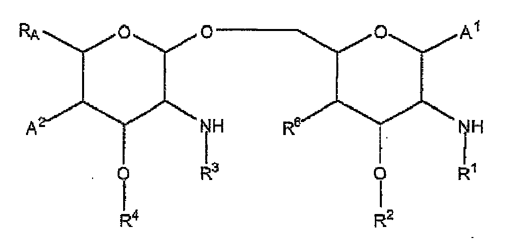 where R¹ is selected from the group consisting of 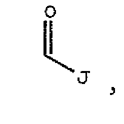 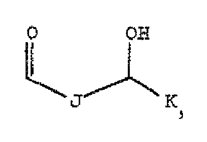 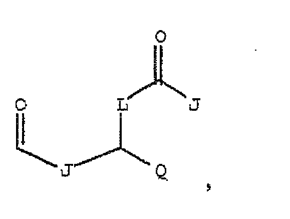 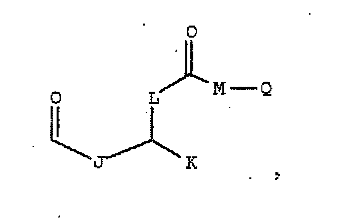 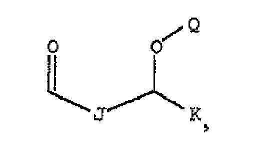 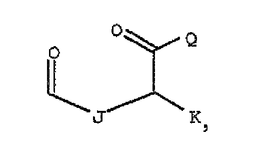 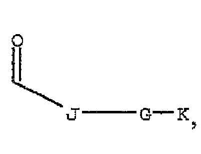 and 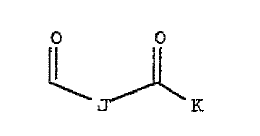 where each of J, K, and Q, independently, is straight or branched C1 to C15 alkyl; L is O, NH, or CH₂; M is O or NH; and G is NH, O, S, SO, or SO₂;
R² is straight or branched C5 to C15 alkyl;
R³ is selected from the group consisting of straight or branched C5 to C18 alkyl, 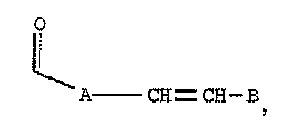 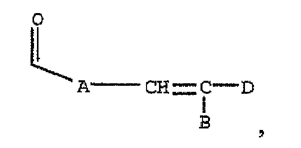 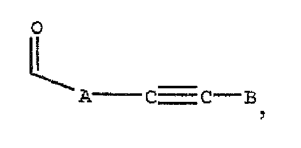 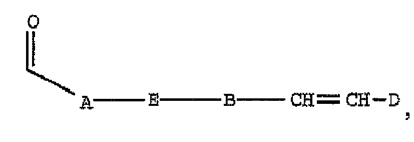 and 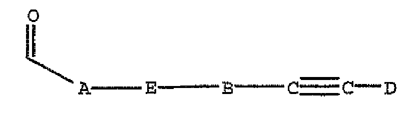
where E is NH, O, S, SO, or SO₂; each of A, B, and D, independently, is straight or branched C1 to C15 alkyl;
R⁴ is selected from the group consisting of straight or branched C4 to C20 alkyl, and 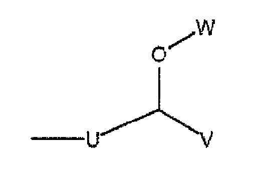
where each of U and V, independently, is straight or branched C2 to C15 alkyl and W is hydrogen or straight or branched C1 to C5 alkyl;
R_{A} is R⁵ or R⁵-O-CH₂-, R⁵ being selected from the group consisting of hydrogen, J', -J'-OH, -J'-O-K', -J'-O-K'-OH, and -J'-O-PO(OH)₂, where each of J' and K', independently, is straight or branched C1 to C5 alkyl;
R⁶ is selected from the group consisting of hydroxy, a halogen, C1 to C5 alkoxy, and C1 to C5 acyloxy;
A¹ and A², independently, are selected from the group consisting of OH, 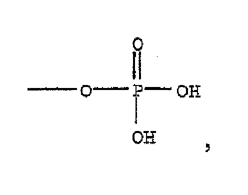 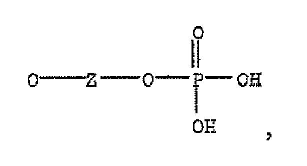 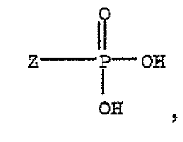 and

   o-z-CO₂H

   where Z is straight or branched C1 to C10 alkyl; or a pharmaceutically acceptable salt thereof.

A specific example of a Lipid A analog that can be use in the methods of the invention is E5564, which has the following structure:

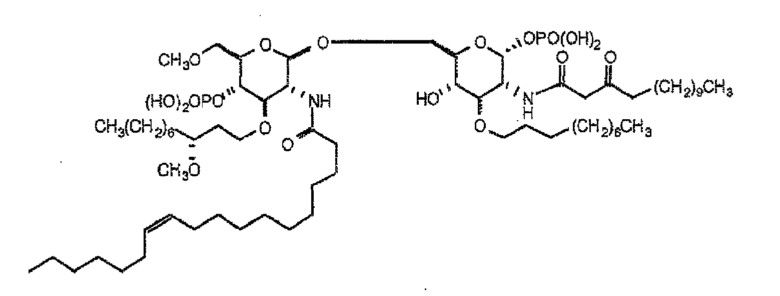

Diseases or conditions that can be treated or prevented using the methods of the invention include, for example, heavy metal poisoning, arthritis, asthma, cardiovascular disease, inflammatory bowel disease, acute renal failure, pancreatitus, infectious disease (e.g., bacterial infection), allograft rejection, graft-versus-host disease, cardiopulmonary bypass-associated morbidity and mortality, side effects of chemotherapy or radiotherapy, mucositis, and autoimmune disease. Specific examples of autoimmune diseases that can be treated or prevented using the methods of the invention include glomerulonephritis, cirrhosis, hepatitis, Addison's Disease, rheumatoid arthritis, systemic lupus erythematosis, pemphigoid, multiple sclerosis, Goodpasture's syndrome, myocarditis, insulin-dependent diabetes, scleroderma, and myasthenia gravis. The invention thus enables the treatment and prevention of a wide variety of diseases and conditions.

The invention also includes use of the Lipid A analogs described herein in the preparation of medicaments for preventing or treating non-sepsis, Toll-like Receptor (TLR)-mediated (e.g., TLR4 or TLR2-mediated), heat shock protein-associated diseases or conditions, such as those described herein.

Other features and advantages of the invention will be apparent from the following detailed description, the drawings, and the claims.

### Brief Description of the Drawings

Fig. 1 is a graph showing the effects of HSP27, HSP40, HSP60, HSP65, HSP90, GroEL, and DNAK on TNF activity in THP1 cells.
Fig. 2 is a graph showing the effects of LPS, HSP60, HSP70, DNAK, and GroEL on IL-6 production in human monocytes.
Fig. 3 is a graph showing the effects of HSP60 and HSP70, with and without heat-inactivation, on IL-6 production in human monocytes.
Fig. 4 is a graph showing the stimulatory effect of HSP60 on TNF production in human monocytes, as well as the impacts of E5564, E5531, their corresponding placebos (Pla1 and Pla2, respectively), and polymyxin B (PMXB) on this effect.
Fig. 5 is a graph showing the stimulatory effect of HSP60 on IL-6 production in human monocytes, as well as the impacts of E5564, E5531, their corresponding placebos (Pla1 and Pla2, respectively), and polymyxin B (PMXB) on this effect.
Fig. 6 is a graph showing the stimulatory effects of LPS and HSP60 on IL-6 production in human chondrocytes, as well as the impacts of heat treatment, polymyxin B (PMXB), and E5564 on these effects.
Fig. 7 is a graph showing the stimulatory effects of LPS and HSP60 on MMP3 production in human chondrocytes, as well as the impacts of heat treatment polymyxin B (PMXB), and E5564 on these effects.
Fig. 8 is a graph showing the stimulatory effects of HSP60 and LPS on IL-6 production in human chondrocytes, as well as the impacts of E5564, E5531, their corresponding placebos (Pla1 and Pla2, respectively), and polymyxin B (PMXB) on these effects.
Fig. 9 is a graph showing the stimulatory effects of HSP60 and LPS on MMP3 production in human chondrocytes, as well as the impacts of E5564, E5531, their corresponding placebos (Pla1 and Pla2, respectively), and polymyxin B (PMXB) on these effects.
Fig. 10 is a graph showing the stimulatory effects of HSP60 and LPS on PGE2 production in human chondrocytes, as well as the impacts of E5564, E5531, their corresponding placebos (Pla1 and Pla2, respectively), and polymyxin B (PMXB) on these effects.
Fig. 11 is a graph showing the stimulatory effects of HSP60 and LPS on IL-8 production in human chondrocytes, as well as the impacts of E5564, E5531, their corresponding placebos (Pla1 and Pla2, respectively), and polymyxin B (PMXB) on these effects.

### Detailed Description

We have shown that heat shock proteins (HSPs) stimulate the production of inflammatory mediators, such as interleukin-6 (IL-6), tumor necrosis factor-α (TNFα), matrix metalloproteinase 3 (MMP3), COX-2-dependent prostaglandin E2 (PGE2), and interleukin-8 (IL-8), and that this stimulatory activity is not due to contaminating lipopolysaccharide (LPS). We have also shown that Lipid A analogs competitively inhibit this activity of HSPs. The invention, thus, provides methods of treating or preventing Toll-like Receptor (TLR), HSP-associated diseases and conditions by administration of Lipid A analogs.

Diseases and conditions that can be treated or prevented using the methods of the present invention are those having a pathogenesis in which HSPs play a role (e.g., a primary role, by, for example, binding to a cell receptor). Examples of such diseases and conditions include heavy metal-induced diseases (e.g., lead, zinc, and cadmium poisonings), arthritis (e.g., septic arthritis), asthma, cardiovascular diseases (e.g., arteriosclerosis, such as atherosclerosis, and hypertension), inflammatory bowel disease (e.g., ulcerative colitis or Crohn's disease), renal vascular disease (e.g., acute renal failure), pancreatitus, infectious disease (e.g., bacterial infection), graft rejection, graft versus host disease, cardiopulmonary bypass-associated morbidity and mortality, and side effects of chemotherapy or radiotherapy (e.g., mucositis). Additional examples of diseases and conditions that can be treated or prevented using the methods of the invention are autoimmune diseases such as, for example, glomerulonephritis, cirrhosis, hepatitis, Addison's Disease, rheumatoid arthritis, systemic lupus erythematosis, pemphigoid, multiple sclerosis, Goodpasture's syndrome, myocarditis, insulin-dependent diabetes, scleroderma, and myasthenia gravis.

Lipid A analogs that can be used in the methods of the invention include, for example, Lipid A analogs, such as Compound E5564 (SGEA; 1287; U.S. Patent No. 5,935,938; see structure, above) and Compound E5531 (U.S. Patent No. 5,530,113), as well as other compounds that are described in these patents and the following U.S. patents: U.S. Patent No. 5,612,476, U.S. Patent No. 5,756,718, U.S. Patent No. 5,843,918, U.S. Patent No. 5,750,664, and U.S. Patent No. 5,681,824, each of which is incorporated herein by reference.

Administration of a Lipid A analog in the methods of the invention can be carried out using any of several standard methods including, for example, continuous infusion, bolus injection, intermittent infusion, inhalation, or any appropriate combinations of these methods. For example, a mode of administration that can be used is continuous intravenous infusion. In such an approach, the infusion dosage rate of the drug can be, for example, 0.001-0.5 mg/kg body weight/hour, more preferably 0.01-0.2 mg/kg/hour, and most preferably 0.03-0.1 mg/kg/hour, infused over the course of, for example, 12-100, 60-80, or about 96 hours. The infusion of the drug can, if desired, be preceded by a bolus injection; preferably, such a bolus injection is given at a dosage of 0.001-0.5 mg/kg. Preferably, the total amount of drug administered to a patient is 25-600 mg, more preferably 35-125 mg, by infusion over a period of 60-100 hours. As activity in the hospital, and particularly the intensive care unit, where this drug may be administered in this manner, is often hectic, minor variations in the time period of infusion of the drug may occur.

Additional modes of administration of E5564, according to the methods of the invention, include bolus or intermittent infusion. For example, the drug can be administered in a single bolus by intravenous infusion through, for example, a central access line or a peripheral venous line, or by direct injection, using a syringe. Such administration may be desirable if a patient does not need prolonged persistence of the drug. In these patients, a single bolus infusion of, e.g., 0.10-15 mg/hour (e.g., 1-7 mg/hour or 3 mg/hour) of drug can be administered over a period of four hours. (Note that the amount of drug administered is based on an assumed average weight of a patient of 70 kg.) Shorter or longer time periods of administration can be used, as determined to be appropriate by one of skill in this art.

In cases in which longer-term persistence of active drug is desirable, intermittent administration can be carried out. In these methods, a loading dose is administered, followed by either (i) a second loading dose and a maintenance dose (or doses), or (ii) a maintenance dose or doses, without a second loading dose, as determined to be appropriate by one of skill in this art. The first (or only) loading dose can be administered in a manner similar to that described for the single bolus infusion described above. That is, for E5564 administration, 0.10-15 mg/hour (e.g., 3-7 mg/hour or 3 mg/hour) of drug can be administered to a patient over a period of four hours. If a second loading dosage is to be used, it can be administered about 12 hours after the initial loading dose, and can involve infusion of, e.g., 0.10-15 mg/hour (e.g., 1-7 mg/hour or 3 mg/hour) of drug over a period of, e.g., about two hours.

To achieve further persistence of active drug, a maintenance dose (or doses) of drug can be administered, so that levels of active drug are maintained in the blood of a patient. Maintenance doses can be administered at levels that are less than the loading dose(s), for example, at a level that is about 1/6 of the loading dose. Specific amounts to be administered in maintenance doses can be determined by a medical professional, with the goal that drug level is at least maintained. Maintenance doses can be administered, for example, for about 2 hours every 12 hours beginning at hour 24 and continuing at, for example, hours 36, 48, 60, 72, 84, 96, 108, and 120. Of course, maintenance doses can be stopped at any point during this time frame, as determined to be appropriate by a medical professional.

In the case of pulmonary diseases or conditions, administration of the compositions of the invention can be effected by means of periodic bolus administration, by continuous, metered inhalation, or by a combination of the two. A single dose is administered by inhalation 1 µg-24 mg, for example, 5-150 µg, or, preferably, 10-100 µg of the drug. Of course, recalcitrant disease may require administration of relatively high doses, e.g., 5 mg, the appropriate amounts of which can be determined by one of skill in this art. Appropriate frequency of administration can be determined by one of skill in this art, and can be, for example, 1-4, for example, 2-3, times each day. Preferably, the drug is administered once each day. In the case of acute administration, treatment is typically carried out for periods of hours or days, while chronic treatment can be carried out for weeks, months, or even years.

Both chronic and acute administration can employ standard pulmonary drug administration formulations. Administration by this route offers several advantages, for example, rapid onset of action by administering the drug to the desired site of action, at higher local concentrations. Pulmonary drug formulations are generally categorized as nebulized (see, e.g., Filament et al., Drug Development and Industrial Pharmacy 21(20):2263-2285,1995) and aerosolized (Sciarra, "Aerosols," Chapter 92 in Remington's Pharmaceutical Sciences, 16th edition (ed. A. Osol), pp. 1614-1628, Malcolmson et al., PSTT 1(9):394-398, 1998, and Newman et al., "Development of New Inhalers for Aerosol Therapy," in Proceedings of the Second International Conference on the Pharmaceutical Aerosol, pp. 1-20) formulations.

For use in the methods of the present invention, a Lipid A analog is formulated with a pharmaceutically acceptable carrier or diluent. Standard method for preparing and formulating drugs are well known in the art and can be used in the invention. For example, the drug, which can be stored as a freeze-dried preparation, can be dissolved in physiological saline (which may include 5% glucose). Alternatively, the drug can be formulated by dissolving 35.4 mg of drug substance in 52.1 ml 0.01 N NaOH, stirring for one hour at room temperature, and diluting into phosphate-buffered lactose. After adjusting the pH to 7.3 and diluting the drug to a final concentration of 0.1 mg/ml, the solution can be filter-sterilized and lyophilized. An example of a formulation of drug product in 1 ml vials is shown below.

**Table 1**

| Material | Amount |
|---|---|
| E5564 | 10 mg |
| NaH₂PO₄· 4H₂O | qs |
| NaOH | qs |
| Lactose hydrous | 400 mg |
| Na₂HPO₄· H₂O | 1.8 mg |
| sterile water | 4 ml |

Additional appropriate formulations can readily be determined by those of skill in this art (see, e.g., Remington's Pharmaceutical Sciences (18th edition), ed. A. Gennaro, 1990, Mack Publishing Company, Easton, PA).

### Summary of Experimental Results

We have found that HSP60, HSP70, and other HSPs significantly increase IL-6 production in human monocytes and TNFα transcription, as indicated by the activity of a reporter gene, placental alkaline phosphatase (PLAP), in THP1 cells. The stimulating activity of HSPs was observed in primary cultures of human chondrocytes as well. We also found that the Lipid A analogs E5564 and E5531 were able to inhibit the stimulatory activities of HSPs. While polymyxin B markedly inhibited LPS induction of IL-6, it did not affect IL-6 production in HSP60 and HSP70-activated human monocytes, showing that the stimulating activity of HSPs is not attributable to LPS contamination. Further, heat treatment significantly decreased the activating activity of HSP60 and HSP70, but not LPS, in human monocytes and human chondrocytes. These results show the stimulating effects of HSPs on macrophage and chondrocyte activation, and demonstrate the antagonistic effects of E5564 and E5531 on such activation. LPS antagonists of the E5564 and E5531 class thus can be used in the treatment and prevention of HSP-associated pathological conditions. Details of the experiments described above are provided as follows.

### Materials and Methods

Human monocytes were isolated and purified from fresh blood by density gradient centrifugation through Ficoll and by adherence. Cells were cultured overnight in RPMI 1640 medium supplemented with 10% heat-inactivated human serum or fetal bovine serum prior to the experiment. THP1 cells that were stably transfected with a TNF-PLAP expression plasmid were cultured in RPMI 1640 medium supplemented with 10% heat-inactivated fetal bovine serum (FBS). Human chondrocytes were maintained in monolayer cultures and differentiated in three-dimensional cultures with Alginate beads for more than three weeks before being used in experiments.

To examine whether E5564 or E5531 inhibits the stimulating activity of HSPs on THP1 cells, human monocytes, and human chondrocytes, heat shock proteins or freshly sonicated LPS (control) were added to cell cultures in the presence or absence of drug (10 mg/ml, in most cases) or polymyxin B (PMXB, 10-20 µg/ml). Compounds E5564 and E5531 were also used in experiments employing human chondrocytes. The supernatants were collected for ELISA analysis or TNF-PLAP assays to test for levels of inflammatory factors, including TNF, IL-6, IL-8, PGE-2, and MMP3.

### Results

### Macrophage and Monocyte Activation bv HSPs

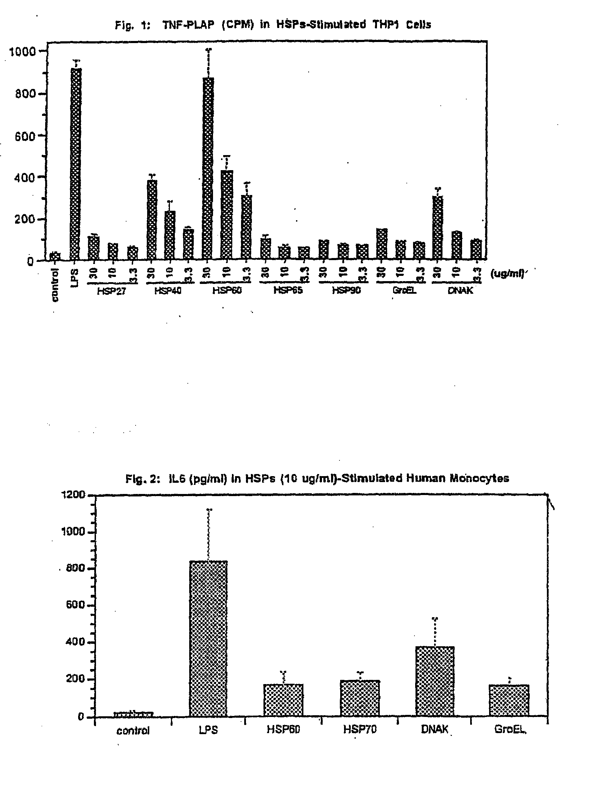

To examine the ability of heat shock proteins to activate macrophage, we examined the effects of some commercially available HSPs on THP1 cell activation. Induction of TNF-PLAP activity was observed in THP1 cells treated with all tested heat shock proteins, as compared with a blank control (Fig. 1). Marked induction of TNF-PLAP activity was observed in THP1 cells treated with HSP60, HSP70, and their bacterial homologs, GroEL/DNAK, as well as HSP40.

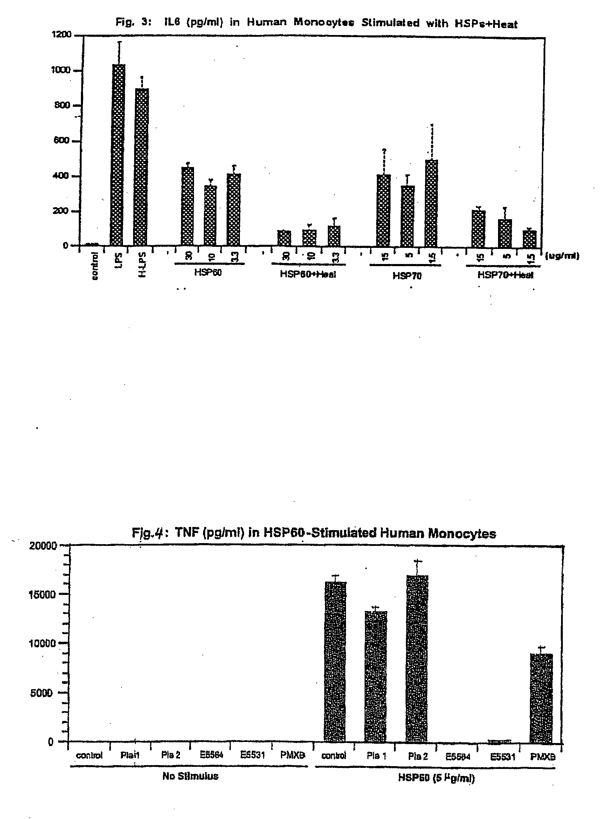

The stimulating activities of HSP60, HSP70, GroEL, and DNAK were also examined in freshly purified human monocytes. IL-6 production was significantly increased in human monocytes treated with heat shock proteins, as compared with a blank control (Fig. 2). Heat treatment of HSP60 and HSP70 significantly decreased their abilities to induce IL-6 production in human monocytes, but failed to inactivate the stimulating activity of LPS (Fig. 3), showing that the stimulating activity of HSP60 and HSP70 on human monocytes is not due to contaminating LPS.

### E5564 Inhibition of TNF and IL-6 Production in HSP-Activated Human

### Macrophages and Monocytes

We next investigated whether the LPS antagonist E5564 would inhibit the stimulating activity of heat shock proteins on THP1 cells and human monocytes. As is shown in Table 2, heat shock proteins increased TNF-PLAP activity in THP 1 cells at different levels. Polymyxin B partially inhibited FLAP levels, and E5564 treatment further decreased TNF-PLAP activity in HSP-treated THP1 cells. In human monocytes, E5564 completely blocked IL-6 release in cells treated with 10-30 µg/ml of HSP60, HSP70, DNAK, or GroEL (Table 3). Polymyxin B had no effect on IL-6 production in HSP60 and HSP70-activated human monocytes, while it completely suppressed LPS-induced IL-6 production. These results demonstrate the inhibitory effect of E5564 on HSP activity.

**Table 2: E5564 Inhibition of TNF-PLAP (cpm) in HSP-Activated THP1 Cells**

| HSPs µg/ml | | HSPs Alone | TNF-PLAP+PMXB | +PMXB +E5564 |
|---|---|---|---|---|
| HSP27 | 30 | 108 | 66 | 37 |
| | 10 | 75 | 79 | 42 |
| | 3.3 | 62 | 49 | 51 |
| HSP40 | 30 | 382 | 148 | 56 |
| | 10 | 234 | 113 | 54 |
| | 3.3 | 143 | 109 | 50 |
| HSP60 | 30 | 2655 | 867 | 336 |
| | 10 | 1079 | 425 | 122 |
| | 3.3 | 520 | 301 | 93 |
| HSP65 | 30 | 92 | 54 | 48 |
| | 10 | 57 | 49 | 51 |
| | 3.3 | 56 | 45 | 54 |
| HSP90 | 30 | 107 | 87 | 53 |
| | 10 | 92 | 65 | 46 |
| | 3.3 | 84 | 64 | 56 |
| GroEL | 30 | 209 | 142 | 82 |
| | 10 | 116 | 90 | 58 |
| 3.3 | 3.3 | 87 | 78 | 61 |
| DNAK | 30 | 698 | 296 | 113 |
| | 10 | 273 | 126 | 81 |
| | 3.3 | 150 | 88 | 66 |
| **Blank** | 0 | 36 | | |
| LPS | 0.1 | 912 | 150 | 60 |

**Table 3: E5564 Inhibition of IL-6 (pg/ml) in HSP-Activated Human Monocytes**

| HSPs | µg/ml | HSPs Alone, | +PMXB | +E5564 |
|---|---|---|---|---|
| HSP60 | 30 | 170 | 225 | 40 |
| | 10 | 170 | 210 | 55 |
| | 3.3 | 245 | 110 | 30 |
| HSP70 | 30 | 225 | 290 | 35 |
| | 10 | 185 | 160 | 30 |
| | 3.3 | 310 | 80 | 30 |
| DNAK | 30 | 430 | 105 | 40 |
| | 10 | 370 | 55 | 60 |
| | 3.3 | 255 | 30 | 75 |
| GroL | 30 | 320 | 30 | 30 |
| | 10 | 160 | 25 | 30 |
| | 3.3 | 160 | 25 | 35 |
| Blank | 0 | 46 | | |
| LPS | 0.1 | 840 | 65 | ND |

### E5531 Inhibition of TNF and IL-6 Production in HSP60-activated Human Monocytes

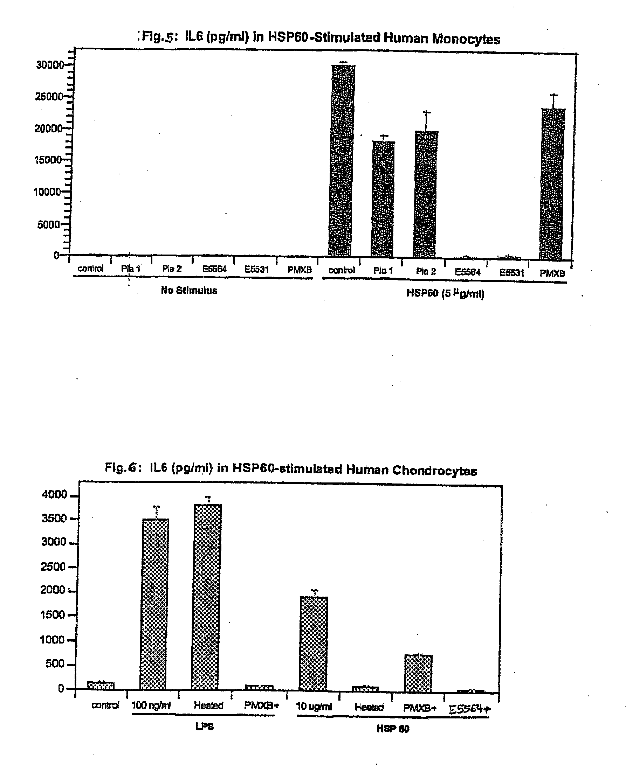

The effect of heat shock protein 60 on monocyte activation was also examined in the absence or presence of E5531 and other agents. As is shown in Figs. 4 and 5, heat shock protein 60 at 5 µg/ml significantly increased TNF and IL-6 production in human monocytes (bar 7), while TNF and IL-6 were not significantly expressed in unstimulated cells (bar 1) and in cells treated with E5531, E5564, or their placebo controls, placebo 1 (Pla1 for E5564) and placebo 2 (Pla2 for E5531). Consistent with our previous results, polymyxin B (PMXB) did not inhibit the increased production of IL-6 and partially suppressed TNF expression, indicating an LPS-independent stimulating activity of heat shock protein 60. In the presence of 10 µg/ml of E5564, HSP60 stimulation of TNF and IL-6 was inhibited to the basal level. E5531 at 10 µg/ml also suppressed TNF and IL-6 to a level close to the base. Since placebos for E5564 (Pla1) and E5531 (Pla2) did not significantly inhibit TNF expression and only partially decreased the IL-6 level, the inhibitory effect of the formulated E5564 and E5531 can be fully or mostly attributed to the activity of E5564 or E5531.

### E5564 and E5531 Suppression of IL-6, MMP3, PGE2, and IL-8 Synthesis in HSP-Activated Human Chondrocytes

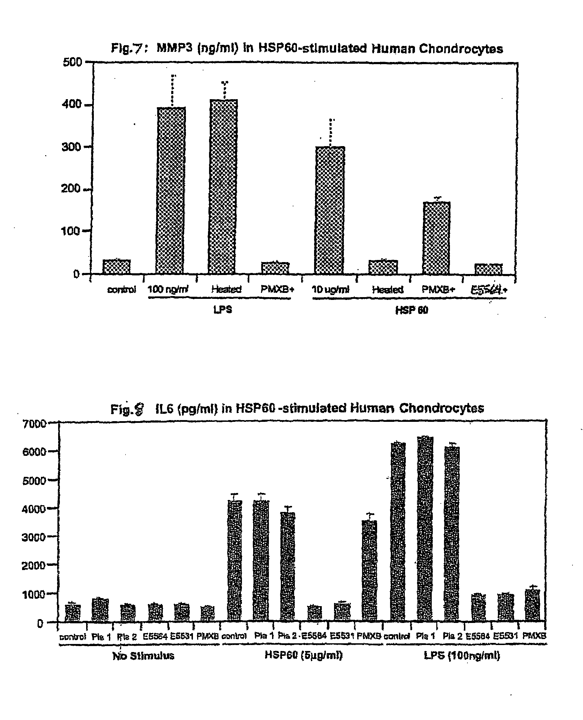

HSP60 activity and the effect of E5564 on HSP60 were examined in primary cultures of human chondrocytes. HSP60 at 10 µg/ml increased IL-6 and MMP3 production in human chondrocytes, as compared with a blank control (Fig. 6 and Fig. 7). Heat treatment significantly decreased IL-6 and MMP3 release in human chondrocytes stimulated with HSP60, but did not affect LPS-induced production of IL-6 and MMP3. Polymyxin B inhibited LPS activity to the basal level and partially suppressed the effects of HSP60. In agreement with the data obtained using human monocytes, the LPS antagonist E5564 completely inhibited IL-6 and MMP3 production in human chondrocytes stimulated with HSP60.

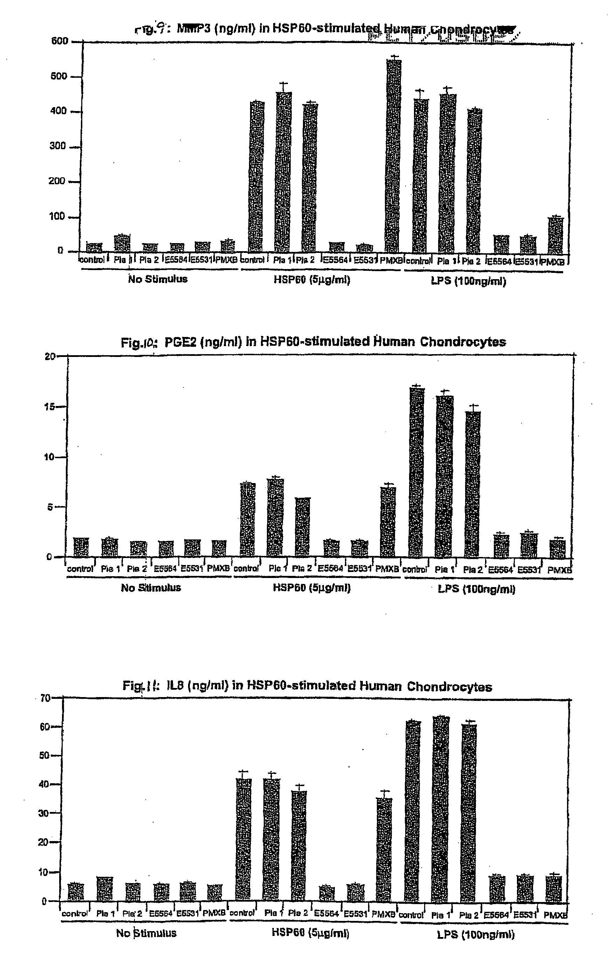

In additional experiments, differentiated human chondrocytes (9F1181) that had been in Aliginate-3D culture for one month were released and plated into 96-well round-bottom plates. One day after plating, the chondrocytes were stimulated with hr-HSP60 or LPS in the presence or absence of E5564 or E5531. After 2 days of culture, conditioned medium was collected and the amounts of IL-6, MMP-3, PGE2, and IL-8 in the medium were measured by ELISA analysis. The results of these experiments are shown in Figs. 8-11. HSP60 at 5 µg/ml increased IL-6, MMP3, PGE2, and IL-8 production (bar 7 of Figs. 8-11) in human chondrocytes as compared to a blank control (bar 1). Neither E5564, E5531, nor their placebo controls (Pla1 and Pla2, respectively) alone stimulated chondrocyte activation. While polymyxin B suppressed LPS-stimulated expression of IL-6, MMP3, PGE2, and IL-8, it did not significantly suppress HSP60 activity.. Similarly, both E5531 and E5564 were able to inhibit the expression of IL-6, MMP3, PGE2, and IL-8 to basal levels. The inhibitory effect must be due to the activities of E5531 and E5564, as the placebos did not significantly decrease the expression of IL-6, MMP3, PGE2, and IL-8 in HSP60-activated chondrocytes.

All patents and publications mentioned above are herein incorporated by reference. Other embodiments are within the following special embodiments.

The invention in particular pertains to the following subject matter:
1. A method of treating or preventing a non-sepsis, Toll-live Receptor-mediated heat shock protein-associated disease or condition in a patient, said method comprising administering to said patient an effective amount of a Lipid A analog of the formula: 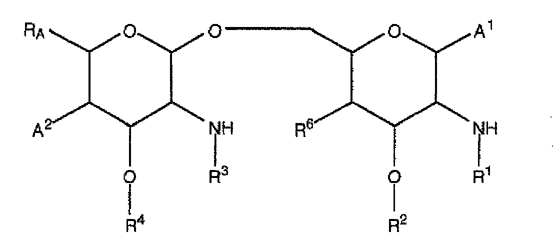 where R¹ is selected from the group consisting of: 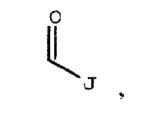 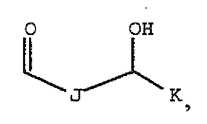 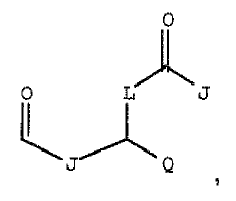 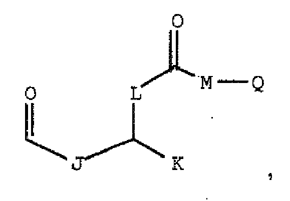 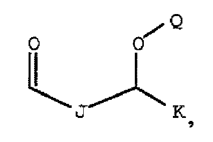 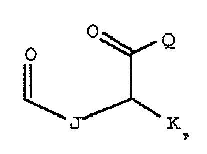 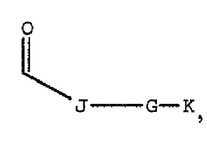 and 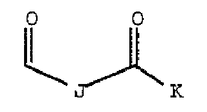 where each of J, K, and Q, independently, is straight or branched C 1 to C15 alkyl; L is O, NH, or CH₂; M is O or NH; and G is NH, O, S, SO, or SO₂;
   R² is straight or branched C5 to C15 alkyl;
   R³ is selected from the group consisting of straight or branched C5 to C18 alkyl, 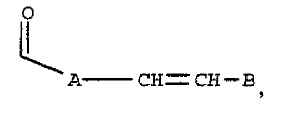 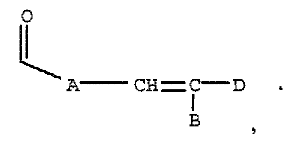 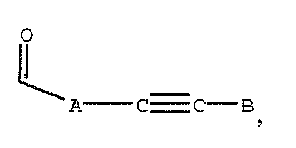 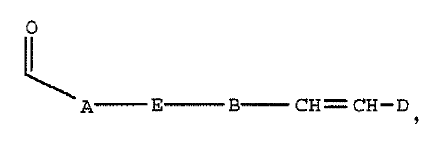 and 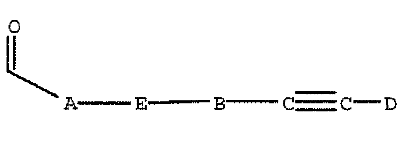
   where E is NH, O, S, SO, or SO₂; each of A, B, and D, independently, is straight or branched C1 to C15 alkyl;
   R⁴ is selected from the group consisting of straight or branched C4 to C20 alkyl, and 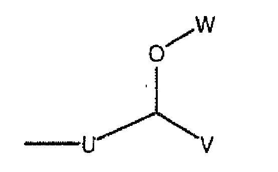
   where each of U and V, independently, is straight or branched C2 to C15 alkyl and W is hydrogen or straight or branched C1 to C5 alkyl;
   R_{A} is R⁵ or R⁵-O-CH₂, R⁵ being selected from the group consisting of hydrogen, J', -J'-OH, -J'-O-K', -J'-O-K'-OH, and -J'-O-PO(OH)₂, where each of J' and K', independently, is straight or branched C1 to C5 alkyl;
   R⁶ is selected from the group consisting of hydroxy, a halogen, C1 to C5 alkoxy, and C1 to C5 acyloxy;
   A¹ and A², independently, are selected from the group consisting of

      OH,

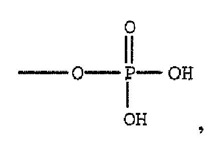

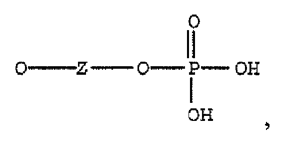

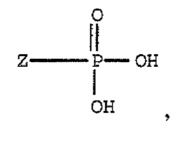

      and

      O-Z-CO₂H

      where Z is straight or branched C1 to C10 alkyl;
      or a pharmaceutically acceptable salt thereof.
2. The method of Item 1, wherein said Lipid A analog has the following structure: 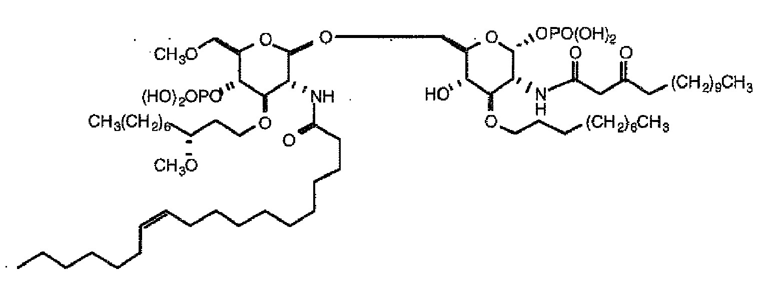
3. The method of Item 1, wherein said disease or condition is selected from the group consisting of: heavy metal poisoning, arthritis, asthma, cardiovascular disease, inflammatory bowel disease, acute renal failure, pancreatitus, bacterial infection, allograft rejection, graft-versus-host disease, cardiopulmonary bypass-associated morbidity and mortality, a side effect of chemotherapy or radiotherapy, mucositis, and an autoimmune disease.
4. The method of Item 3, wherein the disease or condition is an autoimmune disease selected from the group consisting of glomerulonephritis, cirrhosis, hepatitis, Addison's Disease, rheumatoid arthritis, systemic lupus erythematosis, pemphigoid, multiple sclerosis, Goodpasture's syndrome, myocarditis, insulin-dependent diabetes, scleroderma, and myasthenia gravis.
5. The method of Item 1, wherein said Toll-like Receptor is Toll-like Receptor 4.
6. The method of Item 1, wherein said Toll-like Receptor is Toll-like Receptor 2.

## Claims

**1.** Compound selected from the following two formulae E5531 and E5564 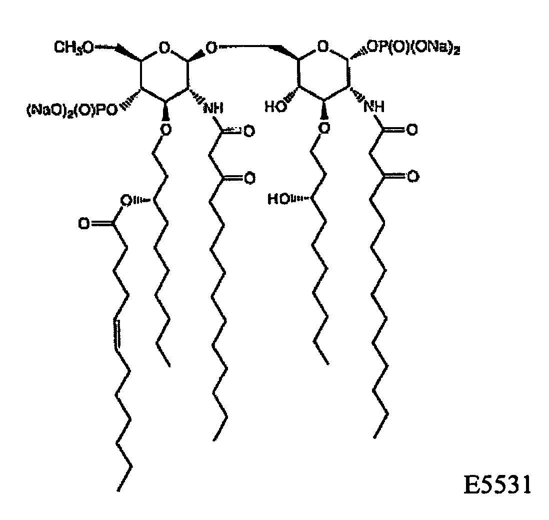 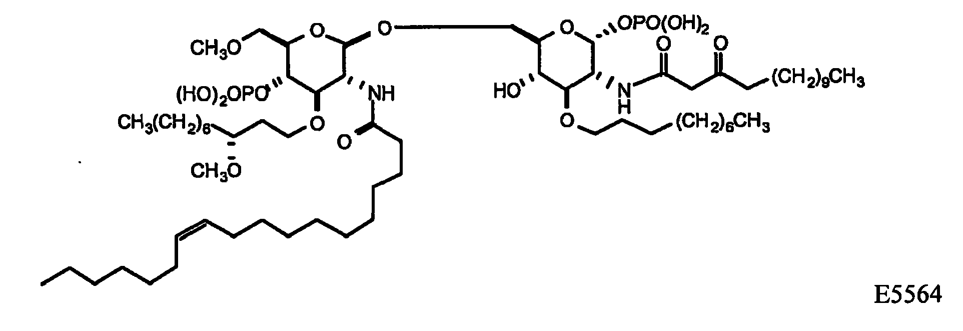 for the treatment or prevention of a non-sepsis, Toll-like Receptor (TLR)-mediated heat shock protein (HSP)-associated disease or condition in a patient.

**2.** The compound for use according to claim 1, wherein the non-sepsis, Toll-like Receptor (TLR)-mediated heat shock protein (HSP)-associated disease or condition is selected from the group consisting of: pancreatitis, cardiovascular disease, ulcerative colitis, Crohn's disease, cardiopulmonary bypass-associated morbidity and mortality, a side effect of chemotherapy or radiotherapy, mucositis, and an autoimmune disease.

**2.** The compound for use according to claim 1, wherein the autoimmune disease is selected from the group consisting of glomerulonephritis, hepatitis, Addison's Disease, rheumatoid arthritis, systemic lupus erythematosis, pemphigoid, multiple sclerosis, Goodpasture's syndrome, myocarditis, insulin-dependent diabetes, scleroderma, and myasthenia gravis.

**3.** The compound for use according to claim 1, 2 or 3, wherein the compound is

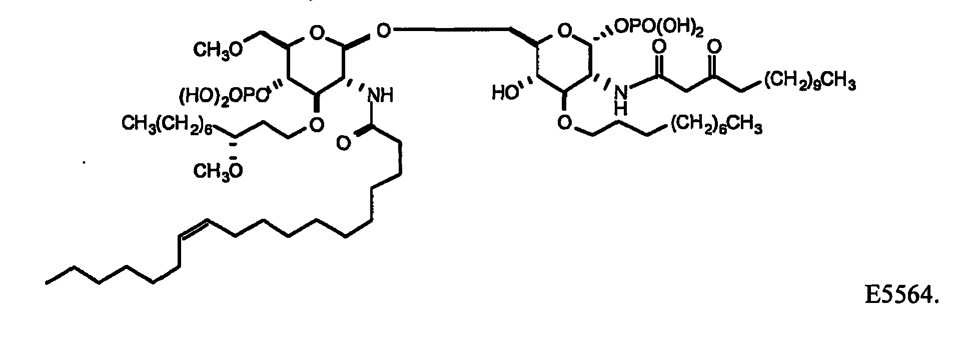

**4.** The compound for use according to any one of claims 1 to 4, wherein the medical indication or condition to be treated or prevented is pancreatitis or hepatitis.
